# EUROPEAN PATENT SPECIFICATION

(11) **EP 2 099 502 B1**
(45) Date of publication and mention of the grant of the patent: **03.03.2010**
(21) Application number: 07853303.1
(22) Date of filing: 07.12.2007
(51) Int. Cl.: A61L 9/12, F21S 10/04, F21S 4/00, F21V 23/04, B05B 17/06

(54) **DIFFUSION DEVICE**
DIFFUSIONSVORRICHTUNG
DISPOSITIF DE DIFFUSION

(30) Priority: 08.12.2006 US 635935
(43) Date of publication of application: 16.09.2009
(73) Proprietor: S.C. Johnson & Son, Inc., Racine, WI 53403 (US)
(72) Inventor: HELF, Thomas A., New Berlin, Wisconsin 53151 (US); PAAS, Edward L., Los Altos, California 94022 (US)
(74) Representative: Ruschke, Hans Edvard
(86) International application number: PCT/US2007/025160
(87) International publication number: WO 2008/073339

(56) References cited:
- US-A1- 2004 264 169
- US-A1- 2005 225 984
- US-A1- 2006 120 080
- US-A1- 2006 125 420

## Description

### BACKGROUND OF THE INVENTION

### 1. Field of the Invention

The present invention relates to diffusion devices, and more particularly, to diffusion devices having a lighting effect.

### 2. Description of the Background of the Invention

Few things are quite as versatile at setting the ambience in an area as scented candles because of their wide array of shapes and sizes, as well as the seemingly limitless number of available scents. However, like any other candle, a scented candle must be used properly and with caution to avoid undesirable consequences.

Electronic lighting devices have been developed that mimic the appearance of a real candle. One device includes two side-by-side lamps that are turned on and off at such frequencies that a flickering effect is perceived. Similarly, another device includes circuitry to control two light bulbs in close proximity to each other such that the bulbs flicker. Moreover, the circuitry and bulbs are held within a container of a size and shape similar to common candles. While these devices may mimic the visual aesthetics of a candle, they fail to provide the scented candle experience, i.e., they fail to emit fragrance in addition to light.

Fragrance dispensers are also generally known. For example, it is known to emit fragrance from an aerosol container upon the activation of a trigger by a user. Also, other methods utilize the evaporative properties of liquids, or other vaporizable materials, to cause vapors with desired properties to be distributed into the ambient air. For example, one fragrance dispenser includes a glass container containing a fluid into which a porous nylon wick extends. The wick extends outwardly from the glass container to disperse fragrance into the surrounding atmosphere with or without the use of a heater, fan, etc. Further volatile releasing devices are disclosed in Denen et al., U.S. Patent No. 6,296,196, filed March 6, 2000, and entitled "Control system for atomizing liquids with a piezoelectric vibrator" and Martens, III, et al., U.S. Patent No. 7,017,829, filed April 14, 2003, and entitled "Atomizer wicking system" the disclosures of which are hereby incorporated by reference herein. Although these devices provide fragrance emission, they do not provide the visual aesthetic of a candle. Document US 2006/120080, on which the preamble of claim 1 is based shows a diffusion device in the form of an artificial candle. It has a flame shaped compartment protuding from the top of a cylindrical housing. The cylindrical housing fits within a cylindrical outer casing with the flame shaped compartment disposed below the top of the casing.

### SUMMARY OF THE INVENTION

According to one aspect of the present invention, a diffusion device is provided as set out in claim 1 below.

Other aspects and advantages of the present invention will become apparent upon consideration of the following detailed description.

### BRIEF DESCRIPTION OF THE DRAWINGS

FIG. 1 is a top isometric view of a first embodiment of a diffusion device;

FIG 2 is an exploded isometric view of the diffusion device of FIG. 1;

FIG. 2A is a bottom isometric view of a top portion of the diffusion device of FIG. 1;

FIG. 3 is a cross-sectional view taken generally along the lines 3-3 of FIG. 1;

FIG. 4 is a view similar to FIG. 1 showing the diffusion device thereof with a top portion removed;

FIG. 5 is a view similar to FIG. 4 with a second support member rotated upwardly to reveal components disposed below the second support member including a printed circuit board;

FIG. 5A is a view similar to FIG. 5 with the printed circuit board removed therefrom;

FIG. 6 is a top isometric view of the diffusion device of FIG. 1 illustrating an opposite side thereof;

FIG. 7 is a bottom isometric view of the diffusion device of FIG. 1 with a bottom portion removed therefrom;

FIG. 8 is an exploded view of a piezoelectric actuator contained within the diffusion device of FIG. 1;

FIG. 9 is a top isometric view of a replaceable fluid reservoir for insertion into any of the embodiments of a diffusion device herein;

FIGS. 10A, 10B, and 10C are elevational views of the diffusion device of FIG. 1 with various interchangeable base portions;

FIG. 11 is an exploded isometric view similar to FIG. 2 of a second embodiment of a diffusion device; and

FIG. 12 is a top isometric view of the diffusion device of FIG. 11 with a second support member rotated upwardly to reveal components disposed below the second support member including a printed circuit board.

Throughout the figures, like or corresponding reference numerals have been used for like or corresponding parts.

### DETAILED DESCRIPTION OF THE PREFERRED EMBODIMENTS

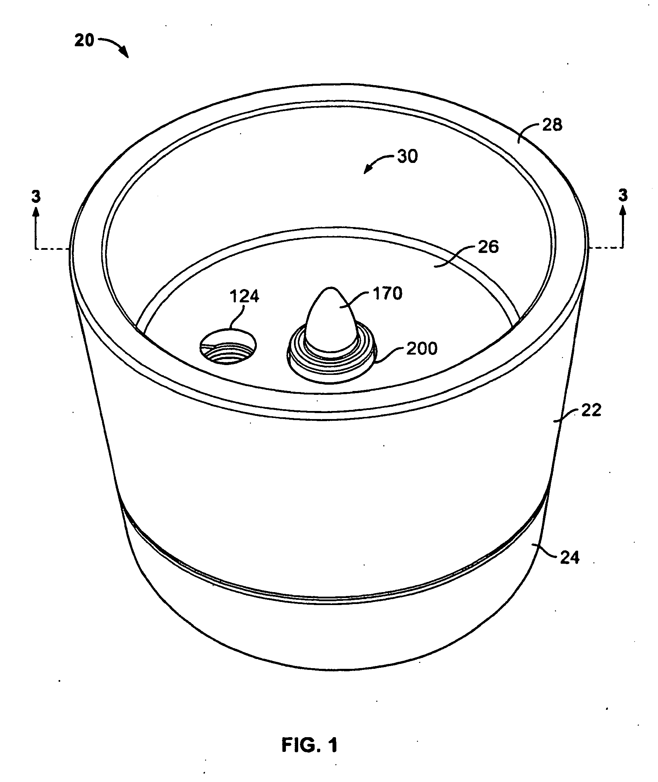

As depicted in FIGS. 1-7, a diffusion device 20 includes a top portion 22 and a bottom base portion 24. The top portion 22 includes a horizontal wall 26 and a cylindrical wall 28 extending upwardly from and horizontal wall 26 to form a cavity 30.

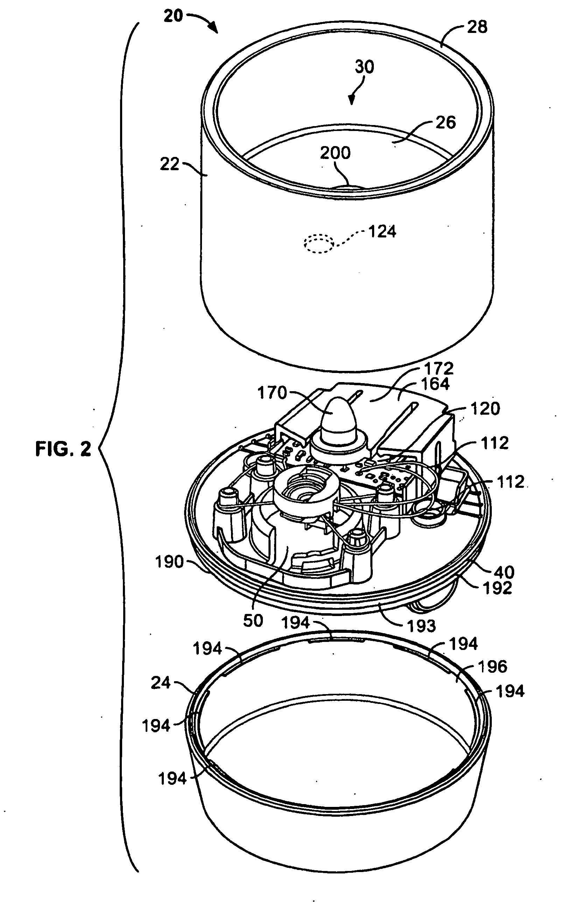

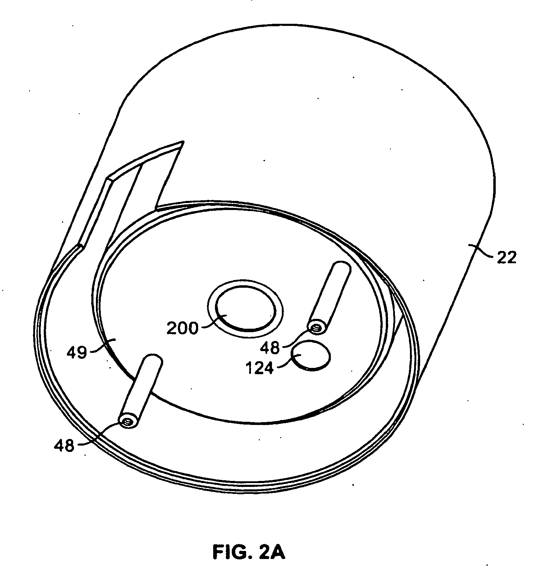

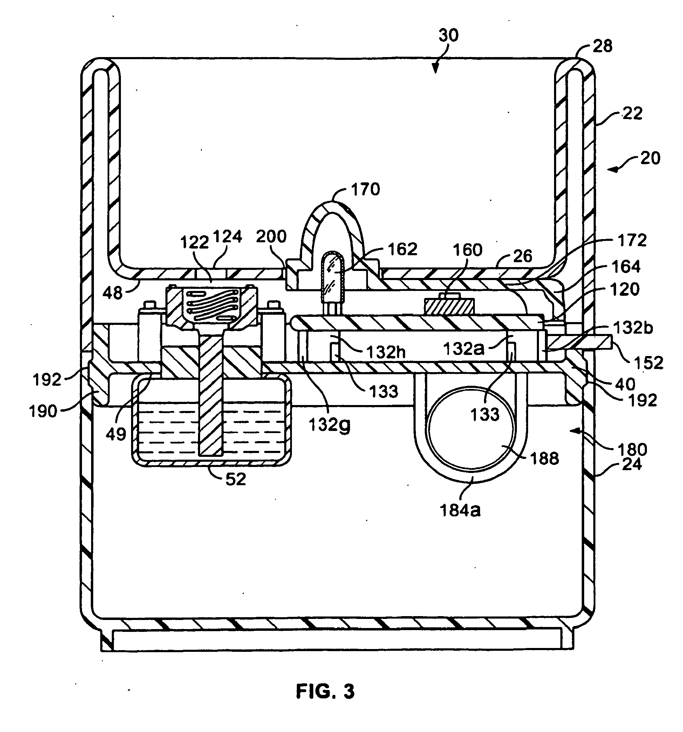

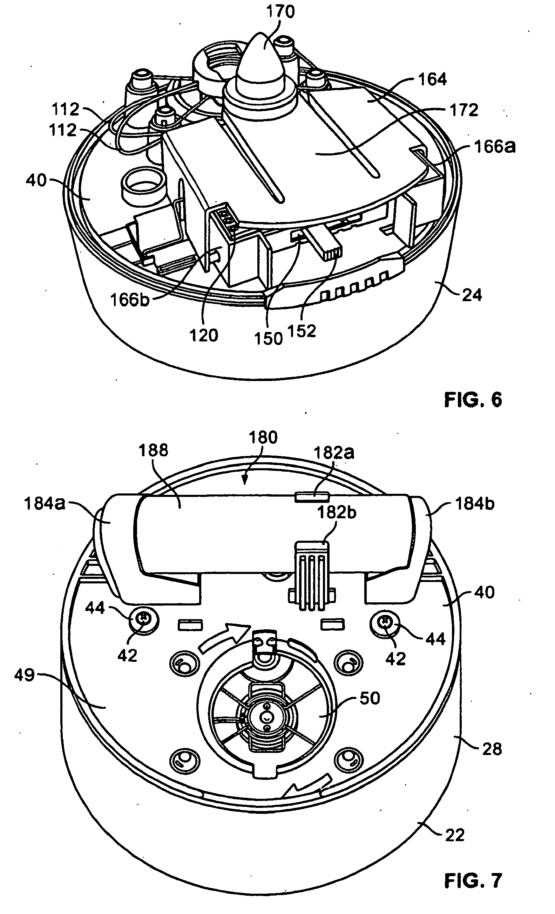

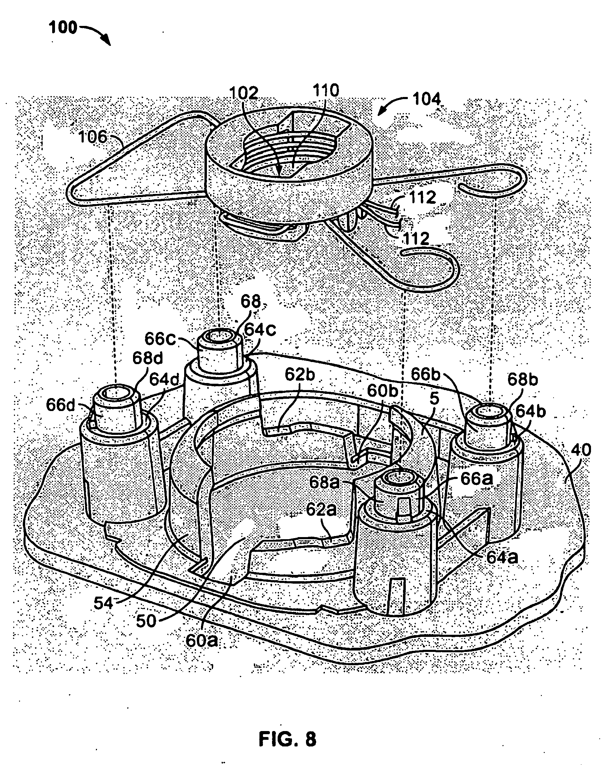

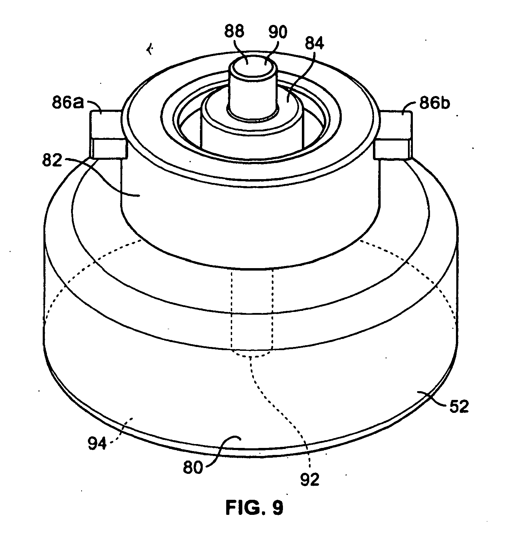

A support member 40 is disposed between the top portion 22 and the base portion 24 and is secured to the top portion 22 by two or more screws 42 that extend upwardly through holes 44 in the support member 40 and into threaded posts 48 that extend downwardly from a bottom surface 49 of the horizontal wall 26 (see, particularly, FIGS. 2A and 7). The support member 40 is formed with an opening 50 therein that receives a replaceable fluid reservoir 52, as seen in FIGS. 3 and 9. As best seen in FIG. 8, a bottle support 54 that forms a part of the support member 40 includes an upwardly extending cylindrically shaped reservoir mounting wall 56. The mounting wall 56 includes two opposing bayonet slots 60a, 60b formed therein and walls 62a, 62b defining corresponding circumferentially extending detents forming a part of the bayonet slots 60a, 60b, respectively. Four cylindrical mounting posts 64a-64d extend upwardly from the support member 40 adjacent the mounting wall 56 wherein each mounting post 64a-64d includes a smaller projection 66a-66d extending upwardly from a top portion 68a-68d thereof. The fluid reservoir 52 is removably inserted into the diffusion device 20, as discussed in detail hereinafter.

The fluid reservoir 52 includes an active material in liquid form therein, wherein the active material is preferably a fragrance. Alternatively, the active material may be an insecticide, an insect repellant, an insect attractant, a disinfectant, a sanitizer, an air purifier, an aromatherapy scent, an antiseptic, an odor eliminator, an air-freshener, a deodorizer, or any other active ingredient(s) that are usefully dispersed into the air.

As shown in FIG. 9, the fluid reservoir 52 comprises a transparent cylindrical container 80 with a neck 82. A combination plug and wick holder 84 is affixed to the neck 82, wherein the plug and wick holder 84 includes a pair of laterally extending mounting lugs 86a, 86b. A wick 88 is disposed within the reservoir 52 in contact with fluid therein. An upper end 90 of the wick 88 extends beyond the neck and a lower end 92 of the wick 88 is disposed within the reservoir 52 toward a bottom surface 94 thereof. The wick 88 transfers liquid by capillary action from within the reservoir 52 to the upper end 90 of the wick 88. The fluid reservoir 52 is inserted into the support member 40 by aligning the lugs 86a, 86b with the bayonet slots 60a, 60b, respectively, and pushing the reservoir 52 upwardly, thereby inserting the lugs 86a, 86b into the respective bayonet slots 60a, 60b. The reservoir 52 is thereafter rotated to force the lugs 86a, 86b to engage with the walls 62a, 62b defining the detent portions of the respective bayonet slots 60a, 60b to secure the reservoir 52 within the diffusion device 20.

Referring next to FIG. 8, a piezoelectric actuator 100 includes a piezoelectric element 102 and orifice plate assembly 104 similar or identical to those described in Denen et al., U.S. Patent No. 6,296,196, filed March 6, 2000, and entitled "Control system for atomizing liquids with a piezoelectric vibrator," Martens, III, et al., U.S. Patent No. 7,017,829, filed April 14, 2003, and entitled "Atomizer wicking system," and Helf et al., U.S. Patent No. 6,896,193, filed November 11, 2002, entitled "Atomizer with improved wire type atomizing element support and method of making same," owned by the assignee of the present application.
The actuator 100 is mounted on the mounting posts 64a-64d by a metal support wire 106 that extends through the actuator 100 and around the mounting posts 64a-64d. The orifice plate assembly 104 comprises an orifice plate 110, wherein an outer circumferential portion of the orifice plate 110 is in contact with the piezoelectric actuator 100. Perforations extend through the orifice plate 110.

The piezoelectric element 102 is connected by wires 112 to a printed circuit board (PCB) 120 (FIGS. 2, 4, 5, 6, and 8), discussed in greater detail hereinafter. The wires 112 supply an alternating voltage produced by circuitry disposed on the PCB 120 to opposite sides of the piezoelectric actuator 100. A diameter of the actuator 100 alternately increases and decreases in size when the alternating voltage is applied to the piezoelectric actuator 100, thereby causing the orifice plate 110 to vibrate up and down due to the contact of the actuator 80 with the orifice plate 110. The orifice plate 110 is, in turn, in contact with fluid supplied by the wick 88. The up and down vibration of the orifice plate 110 causes liquid to be driven through the perforations in the orifice plate 110 and the liquid is emitted upwardly in the form of aerosolized particles. The particles traverse an unobstructed interior 122 (FIG. 3) of the top portion 22 and pass through a first aperture 124 in the horizontal wall 26 of the top portion 22.

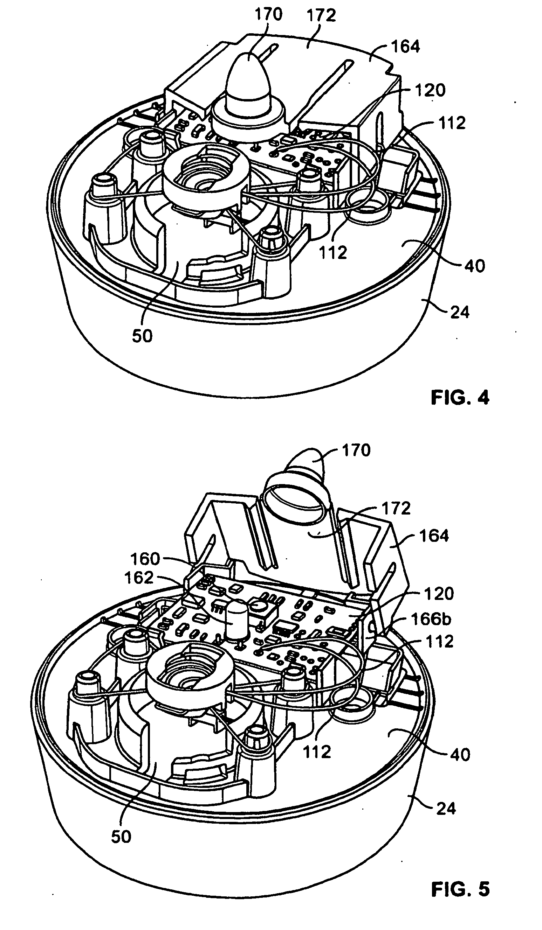

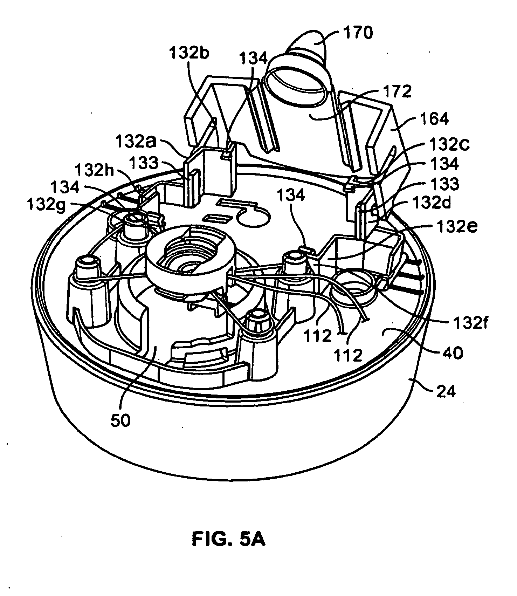

As best seen in FIGS. 3 and 5A, the PCB 120 is mounted on retention members 132a-132h. The retention members 132a, 132d, 132e, and 132h include shoulder portions 133 extending outwardly therefrom at a first height. The retention members 132b, 132c, 132f, and 132g include finger portions 134 extending outwardly therefrom and disposed at a second height that is greater than the first height. The difference between the second and first heights is about the same as a thickness of the PCB 120 such that the PCB 120 snaps between over the shoulder portions 133 to be retained in position between the shoulder portions 133 and the finger portions 134. As seen specifically in FIGS. 3 and 6, the PCB 120 includes a slide switch 150 having a button 152 extending outwardly therefrom. The button 152 is movable to one of five detent positions, which are discussed in greater detail hereinafter. Optionally, the button 152 may be movable to any number of selectable positions. The position of the slide switch 150 is detected by circuitry mounted on the PCB 120 to control the operating mode and emission frequency of the diffusion device 20.

As seen in FIG. 5, a further switch 160 extends upwardly from and is operatively connected to the PCB 120. A light emitting diode (LED) 162 also extends upwardly from and is operatively connected to the PCB 120 and is spaced from the switch 160. A second support member 164 is hingedly connected to the retention members 132a, 132d that aid in supporting the PCB 120 on the support member 40. A hinged, hollow, translucent, and flame-shaped actuator 170 is disposed atop the PCB 120 such that the LED 162 extends into the actuator 170 and a flexure 172 connecting the actuator 170 to the second support member 164 carried by the farst-named support member 40 is in contact with the switch 160. Pressure exerted downwardly on the flame-shaped actuator 170 causes the flexure 172 to trigger the switch 160, as discussed in greater detail hereinafter.

The second support member 164 is hinged to allow the second support member 164 to be rotated from a closed position to an open position. FIG. 5 illustrates the second support member 164 in the open position wherein the PCB 120, switch 160, and LED 162 are accessible and the LED 162 is no longer disposed within the flame-shaped actuator 170. As seen in FIGS. 2 and 4, the PCB 120, switch 160, and LED 162 are not accessible and the flame-shaped actuator 170 is disposed over the LED 162 when the second support member 164 is in the closed position.

As seen in FIGS. 3 and 7, the support member 40 further includes a battery holder 180 including retention fingers 182a, 182b and end contact members 184a, 184b extending from the bottom surface 149 of the support member 40. The battery holder 180 is adapted to receive a single 1.5 volt AA alkaline-manganese dioxide battery 188 and includes contacts for supplying an electrical voltage to the PCB 120. If desired, the single AA battery may be replaced by any number of other batteries or another power source.

The support member 40 also includes an annular plate 190, as seen in FIG. 2, that extends downwardly from the support member 40 away from the top portion 22. The plate 190 includes a continuous radially outwardly-directed flange 192 extending outwardly from the plate 190 that is spaced from the support member 40 to form a channel 195. The base portion 24 includes a plurality of circumferential protrusions 194 extending radially inwardly from an inner surface 196 thereof. The support member 40 fits atop the base portion 24 by snapping the protrusions 194 into the channel 193 to create an interference fit between the protrusions 194 and the flange 192. Optionally, any other known means for attaching two mechanical components may be utilized, such as a bayonet mount, an interference fit, etc.

Referring to FIGS. 1-3, when the support member 40 and top portion 22 are attached, the flame-shaped actuator 170 extends through a second aperture 200 in the horizontal wall 26 forming the top portion 22 and the piezoelectric actuator 100 is disposed below the first-named aperture 124 in the horizontal wall 26. The first-named aperture 124 is positioned such that when the piezoelectric actuator 100 is triggered by the PCB 120, active material in the fluid reservoir 52 is dispensed into the atmosphere through the first-named aperture 124.

In operation, a battery 180 is inserted into the diffusion device 20 to begin operation of the device 20. Thereafter, the user depresses the flame-shaped actuator 170, thereby forcing the flexure 172 downwardly into contact with the button 152 and activating the switch 160. Activation of the switch 160 a first time turns the LED 162 on and also activates a "boost" function. The PCB 120 preferably implements programming to create a flickering effect in the LED 162 when the LED 162 is on and the "boost" function consists of the diffusion device 20 activating the piezoelectric actuator 100 at a rapid pace or in a greater volume to fill the surroundings with the active material. After a "boost" period has expired, the device begins emitting active material in a normal fashion, wherein 12 millisecond bursts - of active material occur at intervals selectable by the slide switch 150. Triggering the switch 160 a second time causes the LED 162 to turn off. Preferably, although not necessarily, the piezoelectric actuator 100 does not turn off.

In one embodiment, the "boost" function consists of emitting active material in a group of ten 12 millisecond pulses, wherein each group of ten pulses lasts for 720 millisecond. Each group of ten 12 millisecond pulses is repeated every sixty seconds. Preferably, the "boost" period is between about 1 minute and about 10 minutes, more preferably between about 2 minutes and about 8 minutes, and most preferably about 5 minutes.

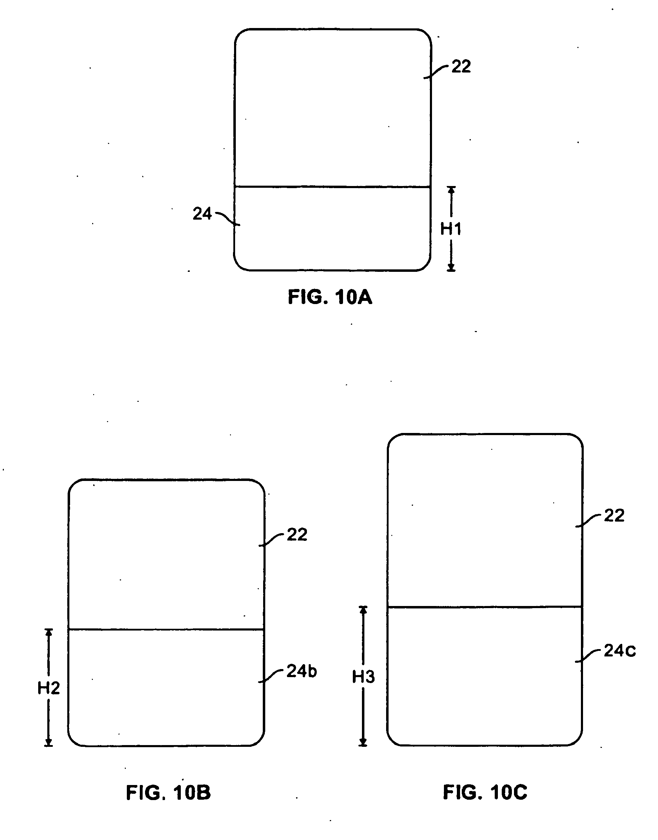

As seen in FIGS. 10A-10C, the top portion 22 is selectively engageable and usable with any of a plurality of base portions 24. In particular, the base portion 24 of FIG. 10A having a height H1 can be used with the top portion 22 or other base portions 24b, 24c may be so used having heights H2, H3, respectively. The heights H1, H2, and H3 are preferably substantially different so that different aesthetics are obtained. Alternatively, different base portions 24 may have different colors, designs, shapes, and/or other features to create different diffusion devices 20. Optionally, the diffusion device 20 may be provided in a kit with one or more base portions 24, such that the diffusion device 20 may be used with any of such base portions 24 to create different imitation candles. In such an embodiment, the kit preferably includes at least one diffusion device 20 and one or more base portions 24 for use with the at least one diffusion device 20. In another embodiment, multiple diffusion devices 20 with a same number of base portions 24 of differing heights could be used to emulate a pillar candle arrangement with multiple candles.

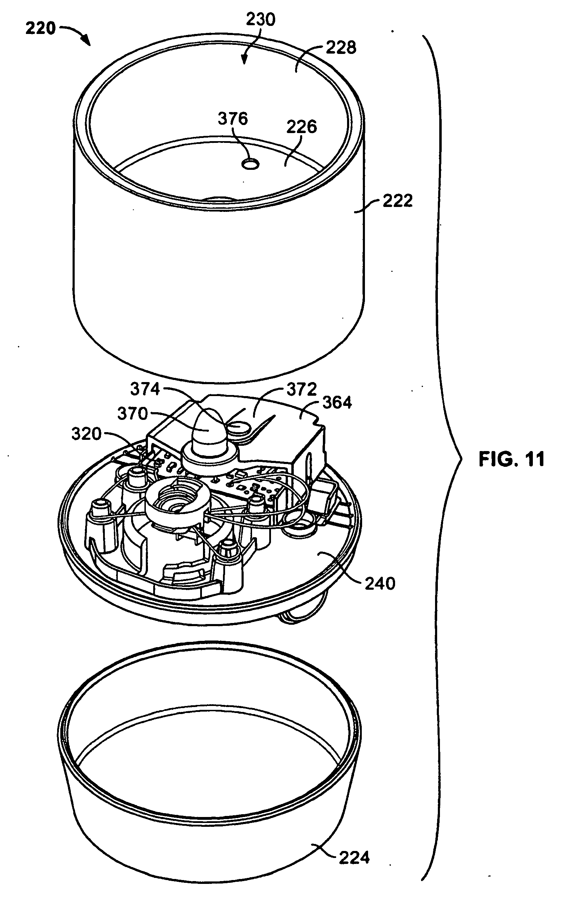

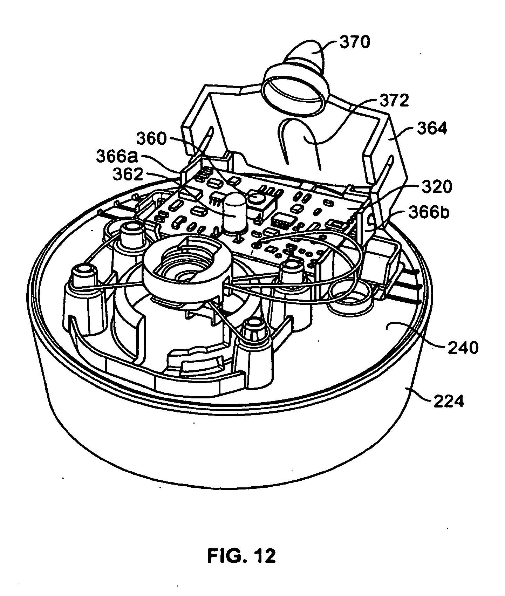

Another embodiment of a diffusion device 220 is depicted in FIGS. 11 and 12. The diffusion device 220 is similar to the diffusion device 20 of FIGS. 1-7, except for the method of activation of the diffusion device 220. In particular, the diffusion device 220 includes a top portion 222 and a bottom base portion 224, wherein the top portion 222 includes a horizontal wall 226 and a cylindrical wall 228 extending upwardly from the horizontal wall 226 to form a cavity 230. The diffusion device 220 includes a support member 240 that is identical to the support member 40 of FIGS. 1-7 and includes the same components attached thereto.

A PCB 320 is supported by the support member 240, as described in detail above, wherein a switch 360 extends upwardly from and is operatively connected to the PCB 320. An LED 362 also extends upwardly from and is operatively connected to the PCB 320 and is spaced from the switch 360. A second support member 364 is hingedly connected to the opposing retention members 366a, 366b. A hollow, translucent, and flame-shaped compartment 370 is disposed atop the PCB 320 such that the LED 362 extends into the compartment 370. A flexure 372 is connected to the second support member 374 and is in contact with the switch 360. The flexure 372 includes a contact member 374 extending upwardly therefrom, wherein the contact member 374 extends through a first aperture 376 in the horizontal wall 226. Pressure exerted downwardly on the contact member 374 causes the flexure 372 to trigger the switch 360. Upon actuation of the switch 360, the LED 362 and the "boost" feature are activated, as discussed in detail above. In all other aspects, the diffusion device 220 is identical to the diffusion device 20 of FIGS. 1-7.

Any of the drive circuits or portions of the drive circuits disclosed in Blandino et al. WO 2008021281 filed August 14, 2006, and entitled "Drive Circuits and Methods for Ultrasonic Piezoelectric Actuators," may be implemented within the PCB 320 of any of the embodiments of diffusion devices disclosed herein.

### INDUSTRIAL APPLICABILITY

The diffusion device of the present invention provides light and/or active material emitters. The device provides an overall desired aesthetic ambience in an area, such as a room.

Numerous modifications to the present invention will be apparent to those skilled in the art in view of the foregoing description. Accordingly, this description is to be construed as illustrative only and is presented for the purpose of enabling those skilled in the art to make and use the invention and to teach the best mode of carrying out same. The exclusive rights to all modifications which come within the scope of the appended claims are reserved.

## Claims

1. A diffusion device (20, 220), comprising:
a housing (22, 24, 222, 224);
a cavity (30, 230) disposed in a top portion (22, 222) of the housing, wherein the cavity is defined by a bottom wall (26, 226);
a flame-shaped compartment (170, 370) extending upwardly through a first aperture (200) in the bottom wall (26) and disposed within the cavity (30, 230);
a second aperture (124) disposed within the bottom wall (26, 226) and spaced from the first aperture (200); and
a substance emitter (100) disposed within the housing (22, 24, 222, 224) wherein the substance emitter (100) is operable to dispense fluid through the second aperture (124);
wherein the diffusion device further includes a bottom portion (24, 224) having a support member (40, 240) that supports a printed circuit board and the substance emitter (100);
a light emitting diode coupled to the printed circuit board and extending into the flame-shaped compartment (170, 370);
**characterized in that** a switch (160, 360) coupled to the printed circuit board wherein the flame-shaped compartment (170, 370) is disposed atop the printed circuit board such that a flexure (172, 372) connecting the flame-shaped compartment (170, 370) to a second support member (164) carried by the first-named support member (40, 240) is in contact with the switch (160, 360).

2. The diffusion device of claim 1, wherein the flame-shaped compartment (170) acts as an actuator in that pressure exerted downwardly on the flame-shaped compartment (170) causes the flexure (172) to trigger the switch (160).

3. The diffusion device of claim 1, wherein a distal end of the flexure (372) includes a contact member (374) that, when pressure is exerted downwardly on the contact member (374), the flexure (372) allows downward movement of the contact member (374) to trigger the switch (360).

4. The diffusion device of claim 2 or 3, wherein activation of the switch (160, 360) a first time turns the light emitting diode on and activates a boost function of the substance emitter (100) and activation of the switch (160, 360) a second time turns the light emitting diode off.

5. The diffusion device of any preceding claim, wherein the housing (22, 24, 222, 224) is cylindrical in shape.

6. The diffusion device of any preceding claim, wherein the substance emitter (100) includes a piezoelectric actuator comprising a piezoelectric element and orifice plate assembly.

7. The diffusion device of any preceding claim, wherein the bottom portion (24, 224) includes a base portion that is removable and replaceable with another base portion.

## Patentansprüche

1. Diffusionsvorrichtung (20, 220), mit:
einem Gehäuse (22, 24, 222, 224);
einem Hohlraum (30, 230), der in einem oberen Abschnitt (22, 222) des Gehäuses angeordnet ist, wobei der Hohlraum durch eine Bodenwand (26, 226) definiert ist;
einer flammenförmigen Kammer (170, 370), die sich nach oben hin durch eine erste Öffnung (200) in der Bodenwand (26) erstreckt und innerhalb des Hohlraums (30, 230) angeordnet ist;
einer zweiten Öffnung (124), die in der Bodenwand (26, 226) angeordnet und von der ersten Öffnung (200) beanstandet ist;
einem Substanzemitter (100), innerhalb des Gehäuses (22, 24, 222, 224) angeordnet ist, wobei der Substanzemitter (100) betrieben werden kann, ein Fluid durch die zweite Öffnung (124) abzugeben;
wobei die Diffusionsvorrichtung weiterhin einen unteren Abschnitt (24, 224) mit einem Stützelement (40, 240) umfasst, das eine Leiterplatine und den Substanzemitter (100) trägt; und mit
einer Leuchtdiode, die mit der Leiterplatine verbunden ist und sich in die flammenförmige Kammer (170, 370) erstreckt;
**dadurch gekennzeichnet, dass** ein Schalter (160, 360) mit der Leiterplatine verbunden ist, wobei die flammenförmige Kammer (170, 370) auf der Leiterplatine angeordnet ist, sodass ein Biegeteil (172, 372), das die flammenförmige Kammer (170, 370) mit einem zweiten Stützelement (164) verbindet, das von dem zuerst genannten Stützelement (40, 240) getragen wird, sich in Kontakt mit dem Schalter (160, 360) befindet.

2. Diffusionsvorrichtung nach Anspruch 1, bei der die flammenförmige Kammer (170) als ein Auslöser derart fungiert, dass die flammenförmige Kammer (170) das Biegeteil (172) veranlasst, den Schalter (160) anzusteuern.

3. Diffusionsvorrichtung nach Anspruch 1, bei der ein distales Ende des Biegeteils (372) ein Kontaktelement (374) umfasst, das dem Biegeteil (372) eine nach unten gerichtete Bewegung des Kontaktelementes (374) gestattet, um den Schalter (360) anzusteuern, wenn auf das Kontaktelement (374) nach unten hin Druck ausgeübt wird.

4. Diffusionsvorrichtung nach Anspruch 2 oder 3, bei der eine erste Aktivierung des Schalters (160, 360) die Leuchtdiode anschaltet und eine Boost-Funktion des Substanzemitters (100) aktiviert und eine zweite Aktivierung des Schalters (160, 360) die Leuchtdiode ausschaltet.

5. Diffusionsvorrichtung nach einem der vorhergehenden Ansprüche, bei der das Gehäuse (22, 24, 222, 224) eine zylindrische Form aufweist.

6. Diffusionsvorrichtung nach einem der vorhergehenden Ansprüche, bei der der Substanzemitter (100) einen piezoelektrischen Aktuator umfasst, der ein piezoelektrisches Element und eine Lochplatten-Baugruppe umfasst.

7. Diffusionsvorrichtung nach einem der vorhergehenden Ansprüche, bei der der untere Abschnitt (24, 224) einen Basisabschnitt umfasst, der entfernbar ist und durch einen anderen Basisabschnitt ersetzbar ist.

## Revendications

1. Dispositif de diffusion (20, 220), comportant :
un boîtier (22, 24, 222, 224) ;
une cavité (30, 230) disposée dans une partie supérieure (22, 222) du boîtier, la cavité étant définie par une paroi de fond (26, 226) ;
un compartiment en forme de flamme (170, 370) s'étendant vers le haut à travers une première ouverture (200) dans la paroi de fond (26) et disposé à l'intérieur de la cavité (30, 230) ;
une deuxième ouverture (124) disposée à l'intérieur de la paroi de fond (26, 226) et espacée de la première ouverture (200) ; et
un émetteur de substance (100) disposé à l'intérieur du boîtier (22, 24, 222, 224), l'émetteur de substance (100) pouvant fonctionner afin de distribuer du fluide à travers la deuxième ouverture (124) ;
le dispositif de diffusion comprenant en outre une partie inférieure (24, 224) ayant un élément de support (40, 240) qui supporte un circuit imprimé et l'émetteur de substance (100) ;
une diode électroluminescente reliée au circuit imprimé et s'étendant dans le compartiment en forme de flamme (170, 370) ;
**caractérisé en ce qu'**un commutateur (160, 360) est relié au circuit imprimé, le compartiment en forme de flamme (170, 370) étant disposé au dessus du circuit imprimé de telle sorte qu'un élément flexible (172, 372) reliant le compartiment en forme de flamme (170, 370) à un deuxième élément de support (164) porté par le premier élément de support (40, 240) est en contact avec le commutateur (160, 360).

2. Dispositif de diffusion selon la revendication 1, dans lequel le compartiment en forme de flamme (170) agit en tant qu'élément d'actionnement de telle sorte qu'une pression exercée vers le bas sur le compartiment en forme de flamme (170) amène l'élément flexible (172) à basculer le commutateur (160).

3. Dispositif de diffusion selon la revendication 1, dans lequel une extrémité distale de l'élément flexible (372) comprend un élément de contact (374) tel que, quand une pression est exercée vers le bas sur l'élément de contact (374), l'élément flexible (372) permet un mouvement vers le bas de l'élément de contact (374) afin de basculer le commutateur (360).

4. Dispositif de diffusion selon la revendication 2 ou 3, dans lequel une activation du commutateur (160, 360) une première fois allume la diode électroluminescente et active une fonction d'accélération de l'émetteur de substance (100) et une activation du commutateur (160, 360) une deuxième fois éteint la diode électroluminescente.

5. Dispositif de diffusion selon l'une quelconque des revendications précédentes, dans lequel le boîtier (22, 24, 222, 224) est d'une forme cylindrique.

6. Dispositif de diffusion selon l'une quelconque des revendications précédentes, dans lequel l'émetteur de substance (100) comprend un dispositif d'actionnement piézoélectrique comportant un ensemble à élément piézoélectrique et plaque à orifice.

7. Dispositif de diffusion selon l'une quelconque des revendications précédentes, dans lequel la partie inférieure (24, 224) comprend une partie de base qui est amovible et remplaçable par une autre partie de base.
